# EUROPEAN PATENT APPLICATION

(11) **EP 4 603 573 A1**
(43) Date of publication of application: **20.08.2025**
(21) Application number: 22962059.6
(22) Date of filing: 13.10.2022
(51) Int. Cl.: C12N 1/00

(54) **METHOD FOR CONTROLLING CULTURE DEVICE, AND METHOD FOR CONTROLLING CULTURE DEVICE IN ASSOCIATION WITH CELL CULTURE**

(71) Applicant: Hitachi, Ltd., Tokyo 100-8280 (JP)
(72) Inventor: SONE, Hiroyuki, Tokyo 100-8280 (JP); ASADA, Hiroyuki, Tokyo 100-8280 (JP)
(74) Representative: MERH-IP Matias Erny Reichl Hoffmann Patentanwälte PartG mbB
(86) International application number: PCT/JP2022/038171
(87) International publication number: WO 2024/079840

(57) **Abstract**

To provide a control method for a culture device which allows culture to be performed in a more suitable culture environment.

A control method for a culture device 1 is a control method for a culture device to input a set variable SV for setting a culture environment and control the culture device performing culture, the control method including: a first step S1 of acquiring, at a predetermined timing, a process variable PV of a culture environment with the set variable SV and an analytical variable AV of a culture solution c obtained by culturing; a second step S2 of deriving by machine learning a function F for calculating a new set variable SV for resetting the culture environment using the process variable PV and the analytical variable AV acquired at the predetermined timing, and a third step S3 of calculating the new set variable SV on the basis of a desired target variable TV of the culture solution by using the function F, inputting the new set variable SV, and performing culture, wherein the function F used in the third step S3 is a new function F derived as needed by machine learning, with a latest process variable PV and a latest analytical variable AV that are acquired at a predetermined timing being included.

## Description

### [Technical Field]

The present invention relates to a control method for a culture device and a control method for a culture device in association with cell culture.

### [Background Art]

Examples of products produced by culture include cosmetics and medicines such as antibodies. For example, when antibody drugs are produced, cells, in which genes producing antibodies are incorporated, are cultured, and antibodies secreted from the cultured cells are purified so as to produce antibody drugs.

Among such products in association with culture, there are some products that require high quality and need to be urgently supplied to the market, such as vaccines.

As a method for controlling the quality of products produced by culture and the like as described above, for example, a method for controlling purification and the like while monitoring quality characteristics and the like of intermediate products in a purification process (downstream process) such as a biological drug purification and concentration process has been proposed (for example, refer to PTL 1).

When such a control method is used, the downstream process is controlled based on purification and concentration process information monitored in real time.

### [Citation List]

### [Patent Literature]

[PTL 1]JP2019-522802A

### [Summary of Invention]

### [Technical Problem]

On the other hand, in a culture process (upstream process) in which cells and microorganisms are proliferated to be cultured, there is still room for more suitable control in response to changes in culture conditions over time that occur due to a cell retention time or the like.

The present invention has been made in view of the above circumstances, and an object of the present invention is to provide a control method for a culture device which allows culture to be performed in a more suitable culture environment and a control method for a culture device in association with cell culture.

### [Solution to Problem]

One aspect of the present disclosure relates to a control method for a culture device to input a set variable for setting a culture environment and control the culture device performing culture, the control method including:
a first step of acquiring, at a predetermined timing, a process variable of a culture environment with the set variable and an analytical variable of a culture solution obtained by culturing;
a second step of deriving by machine learning a function for calculating a new set variable for resetting the culture environment using the process variable and the analytical variable acquired at the predetermined timing; and
a third step of calculating the new set variable on the basis of a desired target variable of the culture solution by using the function, inputting the new set variable, and performing culture,
wherein the function used in the third step is a new function derived as needed by machine learning, with a latest process variable and a latest analytical variable that are acquired at the predetermined timing being included.

In addition, the present disclosure includes a control method for a culture device in association with cell culture using the control method in the production of biopharmaceuticals in association with cell culture.

Here, in this specification, "culture" means proliferation of a single cell or microorganism (unicellular organism, multicellular organism) in a medium. "Updated as needed" includes both a concept of continuous update (update in real time) and a concept of intermittent update at a predetermined interval.

### [Advantageous Effects of Invention]

The present invention can provide a control method for a culture device which allows culture to be performed in a more suitable culture environment and a control method for a culture device in association with cell culture.

### [Brief Description of Drawings]

[Fig. 1]

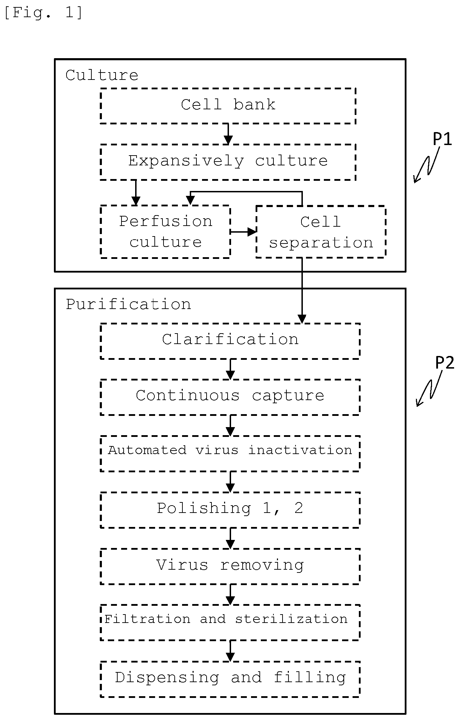

   Fig. 1 is a schematic process diagram showing an example of a production process of producing an antibody drug using a continuous cell culture device.
[Fig. 2]

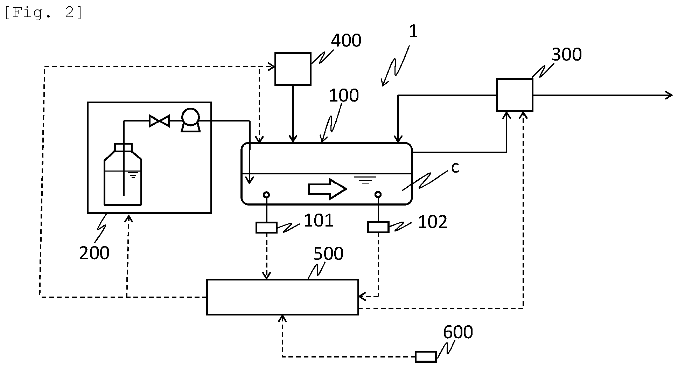

   Fig. 2 is a schematic diagram showing an example of the continuous cell culture device.
[Fig. 3]

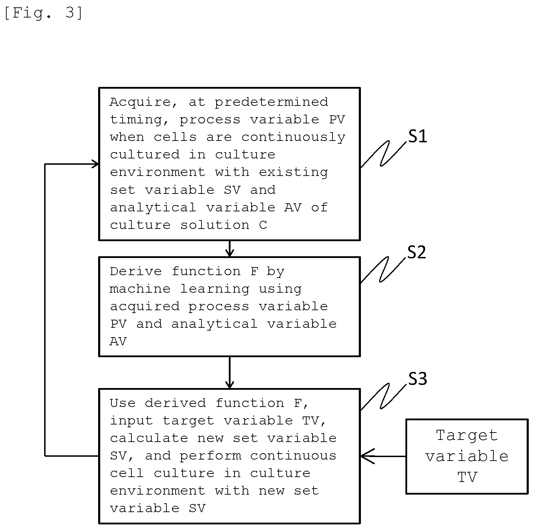

   Fig. 3 is a schematic diagram showing a control method for a culture device according to one embodiment.
[Fig. 4]

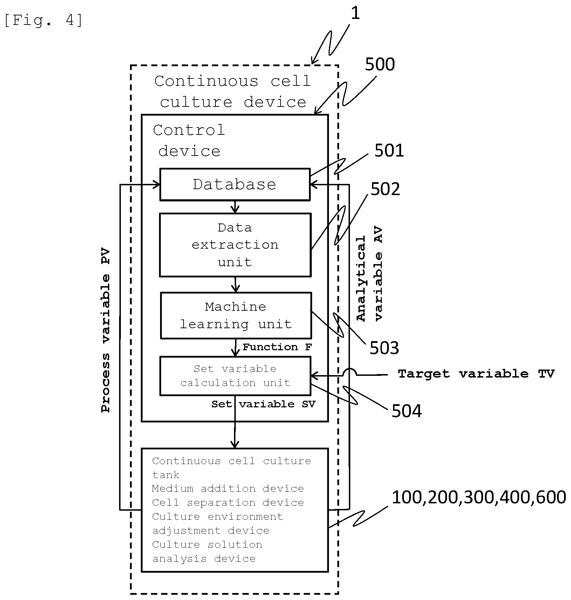

   Fig. 4 is a schematic diagram showing an example of the continuous cell culture device.
[Fig. 5]

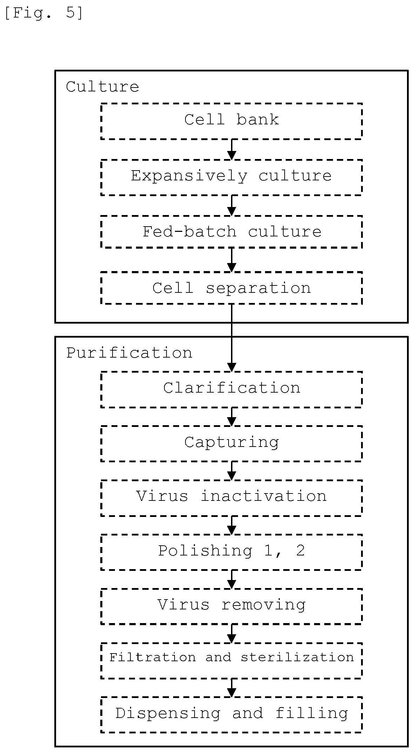

   Fig. 5 is a schematic process diagram showing an example of a production process of producing an antibody drug using a batch type culture device.

### [Description of Embodiments]

### <Control Method for culture device>

A control method for a culture device of the present disclosure is a control method for a culture device including inputting a set variable for setting a culture environment and controlling a culture device that performs culture, the control method including a first step of acquiring, at a predetermined timing, a process variable of a culture environment with the set variable and an analytical variable of a culture solution obtained by culturing, a second step of deriving a function for calculating a new set variable for resetting the culture environment by machine learning using the process variable and analytical variable acquired at the predetermined timing, and a third step of calculating the new set variable based on a desired target variable of the culture solution using the function, inputting the new set variable, and performing culture, wherein the function used in the third step is a new function derived as needed by machine learning, including a latest process variable and a latest analytical variable acquired at the predetermined timing.

Hereinafter, regarding the control method for a culture device according to the present invention, a control method of a cell culture device for culturing cells will be described as an example. Such a control method for a cell culture device can be used, for example, to control a cell culture device that cultures cells and produces drugs such as antibodies. In this specification, a continuous cell culture device is exemplified as the cell culture device. In the continuous cell culture device, a medium is continuously supplied to a culture system, and an equal amount of a culture solution is simultaneously removed from the culture system.

Here, a control method for a culture device to be described below is not limited to control of the cell culture device. In addition, one embodiment of the present invention will be described with reference to the drawings, but the present invention is not limited to the embodiment described in the drawings.

Fig. 1 is a schematic process diagram showing an example of a production process of producing an antibody drug using a continuous cell culture device. As shown in Fig. 1, for example, an antibody drug substance can be produced by executing a culture process P1 and a purification process P2 in this order.

The culture process P1 is a process of culturing (proliferating) cells. The culture process P1 in the present embodiment is performed by controlling a continuous cell culture device. As shown in Fig. 2, a continuous cell culture device 1 can be composed of, for example, a continuous cell culture tank 100, a medium addition device 200, a cell separation device 300, a culture environment adjustment device 400, a control device 500, and a culture solution analysis device 600.

The continuous cell culture tank 100 cultures cells in a culture solution c in a specific culture environment. The sensors 101 and 102 provided in the continuous cell culture tank 100 and the culture solution analysis device 600 measure a PV (Process Variable) and an AV (Analytical Variable) to be described below. The medium addition device 200 stores a fresh medium and supplies the medium to the continuous cell culture tank 100. The cell separation device 300 separates the culture solution and the cells with a membrane, and extracts only the culture solution. The culture environment adjustment device 400 adjusts the culture environment such as the temperature, pH, and dissolved oxygen concentration of the culture solution c. The control device 500 controls the continuous cell culture tank 100, the medium addition device 200, the cell separation device 300, the culture environment adjustment device 400 and the like. Here, the control device 500 will be described below in detail.

The SV (Set Variable) is a variable that sets the culture environment of the continuous cell culture tank 100. The set variables SV are composed of one, two or more parameters. Examples of parameters include a medium supply rate, the temperature, pH, and dissolved oxygen concentration of the culture solution in the continuous cell culture tank 100, a culture solution stirring rate, and a perfusing rate. In order to realize a desired culture environment according to the set variable SV, a temperature adjusting unit for controlling the temperature of the culture solution c, a pH adjusting unit for adjusting the pH of the culture solution c, an oxygen concentration adjusting unit for adjusting the dissolved oxygen concentration in the culture solution c, a stirring unit for uniformly stirring the culture solution c, a perfusion unit for perfusing the culture solution c, and the like, which are not shown, are provided in the continuous cell culture device 1.

The purification process P2 is a process of purifying specific products by separating specific products secreted from cells (physiologically active substance, useful proteins such as antibodies, etc.) and other unnecessary substances (cell shells, viruses, products such as lactic acid and ammonia secreted from cells, etc.) so that the products have a predetermined quality.

In the purification process P2, the purification device (method) used is not particularly limited as long as it can separate out a specific product. Examples of purification devices include a continuous capture device, a virus inactivation device, and a filtration sterilization device, which are shown in Fig. 1, and a gravity precipitation device, an ultrasonic aggregation device, and a filtration separation device, which are not shown. In the purification process P2, these purification devices are used alone or in combination.

Here, in the above purification process P2, publicly known methods and devices can be used.

Here, a control method for the continuous cell culture device 1 used in the culture process P1 among the above processes according to one embodiment will be described with reference to Fig. 3 and Fig. 4.

In the control method for the continuous cell culture device 1, a set variable SV for setting a culture environment is input to control the continuous cell culture device 1. As shown in Fig. 3, the control method for the continuous cell culture device 1 according to the present embodiment includes a first step S1, a second step S2, and a third step S3.

### [First step]

The first step S1 is a step of acquiring, at a predetermined timing, a process variable PV of a culture environment with the set variable SV and an analytical variable AV of a culture solution c obtained by culturing. The first step S1 is executed by measuring the process variable PV and the analytical variable AV by the sensors 101 and 102, the culture solution analysis device 600 and the like.

In the first step S1, specifically, a process variable PV of a culture environment (the temperature, pH, pressure, dissolved oxygen concentration, dissolved carbon dioxide concentration, culture solution volume, and the like of the culture solution c) in the continuous cell culture device 1 operated in a culture environment with a preset set variable SV (hereinafter referred to as an "existing set variable SV") is acquired using the sensor 101 or the like. In addition, in the first step S1, the analytical variable AV, which is an index of quality or productivity (the number of viable cells, the antibody concentration, the impurity concentration, etc.) for the culture solution c cultured by applying the existing set variable SV, is acquired using the sensor 102, the culture solution analysis device 600 or the like. The timing (hereinafter referred to as a "predetermined timing") at which the process variable PV and the analytical variable AV are acquired (actually measured) may be continuous "always" or intermittent "timely." In addition, the process variable PV and the analytical variable AV may be acquired at the same timing or may be acquired at different timings.

### [Second step]

The second step S2 is a step of deriving a function F for calculating a new set variable SV for resetting the culture environment, by machine learning using the process variable PV and analytical variable AV acquired at a predetermined timing. The second step S2 is executed by a database 501, a data extraction unit 502, and a machine learning unit 503 of the control device 500 to be described below.

In the second step S2, specifically, a function F for calculating a new set variable SV is derived by machine learning using the process variable PV and analytical variable AV acquired in the first step S1, which are training data.

Examples of learning methods for machine learning include Bayes estimation, support vector regression, random forest regression, neural networks and the like.

Among these, machine learning is preferably performed using Bayes estimation or support vector regression. When Bayes estimation or support vector regression is applied to machine learning, it is possible to derive a more accurate function F from a relatively small amount of process variable PV and analytical variable AV data that serve as training data. Such learning methods using Bayes estimation or support vector regression are particularly effective methods when there are limited samples during process development (when there is a small amount of available data such as the process variable PV and the analytical variable AV) and when there is an urgent need to supply drugs to the market.

### [Third step]

The third step S3 is a step of calculating a new set variable SV based on a desired TV (Target Variable) of the culture solution c using the function F, inputting the new set variable SV to the continuous cell culture device 1, and performing culture. The third step S3 is executed by a set variable calculation unit 504 (to be described below) of the control device 500, the continuous cell culture tank 100, the medium addition device 200, the cell separation device 300, and the culture environment adjustment device 400.

In the third step S3, first, a new set variable SV is calculated using the desired target variable TV of the culture solution c and the function F derived in the second step S2.

The desired target variable TV of the culture solution c is an index of quality or productivity (the number of viable cells, the antibody concentration, the impurity concentration, etc.).

Here, the new set variable SV may be calculated to be a value within a predetermined range. Specifically, for example, the conditions (setting ranges of parameters to be observed) such as the culture environment in which the continuous cell culture device 1 operates may be determined in advance. In such a case, when the new set variable SV calculated using the function F is a value outside the setting range, the new set variable SV calculated using the function F is corrected to be within the setting range, and the corrected value may be used as the new set variable SV. Thereby, for example, cells can be cultured under approved drug production conditions.

Next, the calculated new set variable SV is input to the continuous cell culture device 1, and culture is performed in the culture solution c in a culture environment of the continuous cell culture device 1 operated using the new set variable SV. That is, culture is performed in the culture solution c under a new culture environment by replacing the existing set variable SV with the new set variable SV. Here, when the value of a parameter constituting the existing set variable SV is the same as the value of a parameter constituting the new set variable SV, the parameter is maintained at the same value.

Here, in the control method for the continuous cell culture device 1, as the function F used in the third step S3, a new function F that is derived as needed by machine learning, including the latest process variable PV and the latest analytical variable AV acquired at a predetermined timing, is used. That is, in the third step S3, the latest function F, which is updated as needed based on the latest information regarding the culture solution c, is used and the new set variable SV is calculated by the latest function F.

The function F may be updated to a new function at the same timing (continuous "always" or intermittent "timely") as the predetermined timing at which the above process variable PV and analytical variable AV are acquired or at a timing different from the predetermined timing. When the function is updated at the same timing as the predetermined timing, culture can be performed in the culture solution c under a culture environment with a new set variable SV obtained using the latest process variable PV and the latest analytical variable AV.

Next, a control device that can execute the above control method for a continuous cell culture device will be described. As shown in Fig. 4, the control device 500 of the continuous cell culture device 1 can be composed of, for example, the database 501, the data extraction unit 502, the machine learning unit 503, and the set variable calculation unit 504.

The database 501 stores set variables SV, process variables PV and analytical variables AV acquired at predetermined timings, and data such as other lots and processes information.

The data extraction unit 502 timely extracts data to be used by the machine learning unit 503 and the set variable calculation unit 504 to be described below, out of data stored in the database 501.

The machine learning unit 503 is a unit that executes step S2. The machine learning unit 503 performs machine learning for deriving a function F for calculating a new set variable SV for resetting the culture environment using the process variable PV and the analytical variable AV acquired from the database 501 through the data extraction unit 502. The process variable PV and the analytical variable AV that serve as training data when machine learning is performed may include the latest process variable PV and the latest analytical variable AV. A more accurate function F can be derived by machine learning using a large amount of training data including the latest information, by including the latest process variable PV and the latest analytical variable AV.

The set variable calculation unit 504 is a unit that calculates a new set variable SV in step S3. The set variable calculation unit 504 calculates a new set variable SV based on a desired target variable TV of the culture solution c using the function F derived by the machine learning unit 503. Information (value) of parameters included in the new set variable SV calculated by the set variable calculation unit 504 is sent to the continuous cell culture tank 100, the medium addition device 200, the cell separation device 300, and the culture environment adjustment device 400.

The above control device 500 can be composed of, for example, the following hardware. That is, the database 501 is composed of a storage device that stores various types of data and the like. The storage device may be composed of any type of storage medium, and may include, for example, a semiconductor memory, a hard disk drive and the like. The data extraction unit 502, the machine learning unit 503, and the set variable calculation unit 504 can be composed of a central processing unit (CPU) that can execute the above processes and the like.

As described above, according to the control method for the continuous cell culture device 1, since the function F used in the third step S3 is a new function F that is derived as needed by machine learning, including the latest process variable PV and analytical variable AV acquired at a predetermined timing, a more accurate new set variable SV can be calculated by machine learning using a larger amount of training data, including the latest information, and culture can be performed in a more suitable culture environment. As a result, it is possible to reliably produce a product having desired quality and productivity.

### <Control Method for culture device in association with cell culture>

In the control method for a culture device in association with cell culture of the present disclosure, the control method for a culture device described above in the section <Control Method for culture device> is used in the production of biopharmaceuticals in association with cell culture.

As in the control method for the continuous cell culture device 1 exemplified in the above embodiment, according to the control method for a culture device of the present disclosure, cells can be cultured in a more suitable culture environment, and biopharmaceuticals such as antibody drugs can be produced with high quality and high productivity. Therefore, the control method for a culture device of the present disclosure can be appropriately applied to the control method for a culture device in association with cell culture used in the production of biopharmaceuticals in association with cell culture.

Here, the present disclosure is not limited to the configurations of the above embodiments, and is defined by the scope of the claims and is intended to encompass equivalents of the scope of the claims and all modifications falling within the scope of the claims. Some of the configurations of the above embodiments may be deleted or replaced with other configurations, and other configurations may be added to the configurations of the above embodiments.

For example, in the above embodiment, the control method for the continuous cell culture device 1, which allows an antibody drug (biopharmaceutical) to be produced, has been exemplified as the control method for a culture device. However, the control method for controlling a culture device of the present disclosure can also be applied to the culture of entities other than cells as long as they can proliferate by culture. The control method for a culture device of the present disclosure can also be applied to, for example, a control method for a culture device which allows cosmetics to be produced by culturing microorganisms such as unicellular organisms and multicellular organisms.

In addition, in the above embodiment, the continuous culture device 1 has been exemplified as the culture device to which the control method for a culture device is applied. However, the control method for a culture device of the present disclosure may be applied to a control method for a culture device that performs a batch process (batch type culture device) as shown in Fig. 5 (Fig. 5 shows an example of a process of producing a biopharmaceutical substance using a batch type cell culture device). In a control method for the batch type culture device, set variables, process variables, analytical variables, target variables, and functions can be handled in the same manner as in the continuous culture device.

### [Reference Signs List]

- 1: Continuous cell culture device (culture device)
- SV: Set variable
- PV: Process variable
- AV: Analytical variable
- TV: Target variable
- S1: First step
- S2: Second step
- S3: Third step
- P1: Culture process
- P2: Purification process

## Claims

1. A control method for a culture device to input a set variable for setting a culture environment and control the culture device performing culture, the control method comprising:
a first step of acquiring, at a predetermined timing, a process variable of a culture environment with the set variable and an analytical variable of a culture solution obtained by culturing;
a second step of deriving by machine learning a function for calculating a new set variable for resetting the culture environment using the process variable and the analytical variable acquired at the predetermined timing; and
a third step of calculating the new set variable on the basis of a desired target variable of the culture solution by using the function, inputting the new set variable, and performing culture,
wherein the function used in the third step is a new function derived as needed by machine learning, with a latest process variable and a latest analytical variable that are acquired at the predetermined timing being included.

2. The control method according to claim 1, wherein the function is updated to a new function at the same timing as the predetermined timing.

3. The control method according to claim 1, wherein the machine learning is performed using Bayes estimation or support vector regression.

4. The control method according to claim 1, wherein the new set variable is calculated so as to be a value within a predetermined range.

5. A control method for a culture device in association with cell culture, the control method comprising using the control method according to any one of claims 1 to 4 in production of biopharmaceuticals in association with cell culture.
